(19) Europäisches Patentamt
European Patent Office
Office européen des brevets

(11) Publication number: **0 129 296**
**B1**

# (12) EUROPEAN PATENT SPECIFICATION

(45) Date of publication of patent specification: **20.01.88**

(51) Int. Cl.[4]: **C 07 D 207/273**

(21) Application number: **84200860.9**

(22) Date of filing: **14.06.84**

## (54) Process for the preparation of N-arylhalopyrrolidones.

(30) Priority: **16.06.83 US 505135**

(43) Date of publication of application:
**27.12.84 Bulletin 84/52**

(45) Publication of the grant of the patent:
**20.01.88 Bulletin 88/03**

(84) Designated Contracting States:
**AT BE CH DE FR GB IT LI NL**

(58) References cited:
**EP-A-0 055 215**
**DE-A-2 612 731**
**US-A-4 132 713**

(73) Proprietor: **STAUFFER CHEMICAL COMPANY**
**Westport Connecticut 06880 (US)**

(72) Inventor: **Broadhurst, Michael David**
**144 Rowland Road**
**Fairfield Connecticut 06430 (US)**
Inventor: **Gless, Richard Douglas, Jr.**
**1918 Rosecrest Drive**
**Oakland California 94602 (US)**

(74) Representative: **Urbanus, Henricus Maria, Ir. et al**
**c/o Vereenigde Octrooibureaux Nieuwe Parklaan 107**
**NL-2587 BP 's-Gravenhage (NL)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

## Description

Background of the Invention

Certain N-arylhalopyrrolidones are known to be useful as herbicides of general application. Such compounds and their utility are disclosed in, for instance, US—A—4,110,105 of Eugene G. Teach.

One process for their production is disclosed in US—A—4,110,105 and additionally US—A—4,210,589, which is a division of the former. According to these references, the compounds are prepared by the intramolecular cyclization of an α-halogen-containing N-2-alkenyl-amide in the presence of a catalytic amount of ferrous iron.

US—A—4,132,713, of Michael D. Broadhurst, discloses an improved method of producing such compounds utilizing catalysts other than ferrous iron. In particular, the catalyst of US—A—4,132,713 contains one or more of the transition metals vanadium, molybdenum, ruthenium, silver, and copper. The metal-containing compounds may be present in a number of forms, including complexes with common complexing agents such as triphenylphosphine, carbon monoxide, and tertiary amines. Examples of tertiary amines disclosed to be useful in that process are pyridine, 2,2'-dipyridyl, 2,2'-dipyridylamine, and tetramethylethylenediamine. Among the copper-containing compounds specifically disclosed in that patent are cuprous chloride and cupric oxide.

When using copper-containing catalysts with tertiary amine complexing agents, the process may be conducted at a temperature of from 60°C to 200°C, preferably from 80°C to 150°C. Table II of US—A—4,132,713 shows the results for production of the compound 3-chloro-4-chloromethyl-1-(m-trifluoromethylphenyl)-2-pyrrolidone,

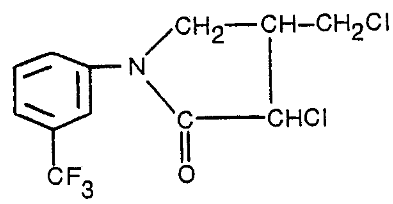

tilizing cupric oxide and cuprous chloride variously complexed with several tertiary amines. The process was conducted at times ranging from 2.5 to 11 hours with yields ranging up to 65% of theoretical. A larger scale preparation of the product is described in Example 14 of the patent. Other examples show production of related compounds by this technique.

A similar process is disclosed in EP—A—0055215 for the production of N-substituted 3-fluoro-4-chloromethyl-2-pyrrolidone compounds, in which the N-substituent may be, among others, a m-trifluoromethylphenyl group. Several catalysts are mentioned, among which a complex of cuprous chloride and piperidine, i.e. a complex formed between a copper compound and a secondary amine.

Summary of the Invention

It has now been found that an improvement with respect to the process generally described in US—A—4,132,713 and in EP—A—0055215 may be obtained by conducting the reaction in the presence of a catalyst comprising a copper compound together with an amine selected from the group consisting of:

(a) primary amines having the formula $RNH_2$, in which R is a straight- or branched-chain alkyl group having from 1 to 20 carbon atoms, optionally substituted by hydroxy or

(b) secondary amines having the formula $R_1NHR_2$, in which $R_1$ and $R_2$ are independently straight- or branched-chain alkyl groups having from 1 to 20 carbon atoms, optionally substituted by hydroxy, exclusive of branched-chain alkyl groups having the branching at the alpha-carbon atom.

Detailed Description of the Invention

The N-2-alkenyl-α-haloamide which is used as a starting material in the process of the present invention may be prepared by any conventional technique known in the art. Several techniques are provided in US—A—4,132,713.

The N-arylhalopyrrolidones produced according to the present invention have the general formula

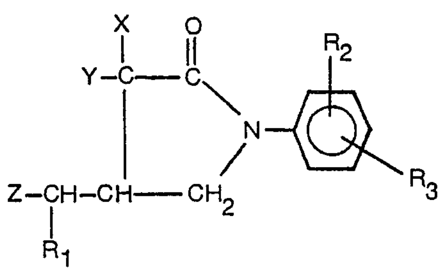

in which

X is hydrogen, chlorine, bromine or fluorine;

Y is hydrogen, chlorine, bromine or fluorine;

Z is chlorine or bromine;

$R_1$ is hydrogen or $C_1$—$C_4$ alkyl;

$R_2$ is hydrogen, $C_1$—$C_4$ alkyl, acetyl, chlorine, bromine, fluorine, iodine, trifluoromethyl, nitro, cyano, $C_1$—$C_4$ alkoxy, $C_1$—$C_4$ alkylthio, $C_1$—$C_4$ alkylsulfinyl, $C_1$—$C_4$ alkylsulfonyl, trifluoromethylthio, trifluoromethylsulfinyl, trifluoromethylsulfonyl, pentafluoropropionamido, or 3-methylureido; and

$R_3$ is hydrogen, $C_1$—$C_4$ alkyl, chlorine, or trifluoromethyl.

A class of preferred products are those in which $R_3$ is trifluoromethyl and $R_2$ is hydrogen or fluoro. One preferred product is 3-chloro-4-chloromethyl-1-(m-trifluoromethylphenyl)-2-pyrrolidone (X = chloro, Y = hydrogen, Z = chloro, $R_1$ = hydrogen, $R_2$ = hydrogen, $R_3$ = 3-trifluoromethyl).

The copper containing catalyst comprises preferably cuprous chloride, cupric chloride or cupric oxide; cuprous chloride being most preferred.

The catalyst can either be present as an undissolved solid in the reaction mixture, or as a solute in solution with the starting amide or solvent, when a solvent is used. In general, the catalyst is preferably dissolved. The reaction will proceed without agitation, whether the catalyst is undissolved or in solution. However, when agitation is used, the progress of the reaction will be significantly enhanced. Agitation may be achieved by any conventional means, for instance stirring, inert gas purging, the use of baffles in the reaction vessel, or conducting the reaction at reflux.

The quantity of copper-containing catalyst which will constitute a catalytic amount will be that quantity which serves to increase the rate of reaction. Larger quantities will provide a greater increase. The quantity used in any particular application will be determined in large part by the individual needs of the manufacturing facility. Aside from considerations such as cost, the desired reaction time and system capacity, the catalyst quantity is not a critical feature in the invention and may vary over a wide range. Most conveniently, an amount of catalyst is used which comprises from 0.1 to 20.0 mole percent, based on the initial quantity of the N-2-alkenylamide.

Preferred quantities of catalyst are: for cuprous or cupric chloride — from 1 to 10 mole percent; for cupric oxide — from 1 to 5 mole percent.

The amines which have been found useful in the improved process of this invention are certain primary and secondary aliphatic amines.

The primary amines are of the type $RNH_2$, in which R is a straight- or branched-chain alkyl group having from 1 to 20 carbon atoms, optionally substituted by hydroxyl. Such amines would include, for instance, ethylamine, propylamines, various butylamines, and amines containing higher alkyl groups. One example of a hydroxy-substituted alkyl amine is 6-hydroxyhexylamine.

The secondary amines are of the $R_1NHR_2$, in which $R_1$ and $R_2$ are alkyl groups having from 1 to 20 carbon atoms, preferably from 1 to 12 carbon atoms. These alkyl groups may be straight- or branched-chain groups, optionally substituted by hydroxy, with the proviso that if branched-chained, the branching is other than at the alpha-carbon atom. Secondary amines containing alpha-branched alkyl groups, such as isopropyl, sec-butyl, and the like, do not provide advantageous results in this process. The alkyl groups $R_1$ and $R_2$ may be the same or different; preferably they are the same. A preferred class of secondary amines are those in which $R_1$ and $R_2$ are identical straight-chain alkyl groups having from 1 to 12 carbon atoms. Preferred members of this class are di(n-butyl) and di-(n-propyl) amines.

If desired, a variety of solvents may be used in the reaction. Such solvents may include aliphatic compounds such as heptane or octane, alcohols such as tertiary butyl alcohol, and aromatic compounds such as toluene or xylene. Preferred solvents are toluene and tertiary butyl alcohol. Other inert solvents such as those mentioned in US—A—4,132,713 may be employed if desired.

The amount of amine used may vary widely according to the costs and effect desired. In general, the amine is utilized in an amount of from 5 to 60 mole percent, based on the starting N-2-alkenyl-α-haloamide, and preferably from 10 to 40 mole percent.

The temperature at which the process is conducted is generally from 50°C to 150°C. Preferred operating ranges will vary, depending on the nature of the copper-containing catalyst, the amine, the solvent (if any), and the concentration of the α-haloamide in the substrate. Generally, cuprous chloride and lower molecular weight amines containing up to eight carbon atoms per alkyl group perform best at temperatures of from 60 to 90°C.

As the reaction occurs in the liquid phase, the operating pressure is not a significant parameter, and may range widely, depending on convenience, economy and materials of construction. It is most convenient to conduct the reaction at or near ambient pressure.

If the reaction is conducted in the presence of a solvent, under reflux or at or near atmospheric pressure, the temperature may be the normal reflux temperature of the solvent, or may be lower. Temperatures of about 85°C have been found advantageous for solvents such as toluene and tertiary butyl alcohol.

The concentration of α-haloamide in the solvent may vary. However, as the concentration is increased from 20 weight % to 60 weight %, selectivity and yield of the reaction generally decrease, with the production of less desired products and greater amounts of "tars". This is particularly true for higher

molecular weight amines and cupric oxide catalysts. For good results, therefore, the concentration of the amide in the solvent should be at a maximum of about 65 weight %, most preferably about 40 weight %.

The order of addition of materials is not a significant parameter. However, it is preferable to pre-mix the copper catalyst and amine, and then add the α-haloamide, as this can produce a small improvement in yield.

A preferred set of parameters for carrying out this process are:

| | |
|---|---|
| copper catalyst | : 3—8 mole % |
| amine | : 20—40 mole % |
| solvent | : toluene |
| temperature | : 75—95°C |
| amide concentration in solvent | : 15—30% |

The pyrrolidone produced by the reaction can be recovered from the reaction mixture by any conventional technique, such as solvent extraction, crystallization, sublimation, or distillation.

As compared with the copper/tertiary amine complexes described in US—A—4,132,713, the use of a copper catalyst with a primary or secondary aliphatic amine of the type described herein results in the production of a greater yield of the desired compound, with a corresponding decrease in the production of non-volatile by-products ("tars"), and generally a decrease in the necessary reaction time.

The following examples illustrate the conduct of the process, with respect to the production of the compound 3-chloro-4-chloromethyl-1-(m-trifluoromethylphenyl)-2-pyrrolidone.

### Example 1

In a flask were mixed 200 grams (g) (0.567 mole) N-allyl-3'-trifluoromethyl-2,2-dichloroacetanilide, 2.79g (0.028 mole) cuprous chloride, 28.5 milliliters (ml) (0.169 mole) di-(n-butyl)amine, and 891 ml toluene. The mixture was heated to a temperature of 85—90°C and stirred for 2 hours and 25 minutes. At the end of this time, a sample of the reaction mixture was analyzed by gas chromatography and showed 95.0 area percent of the desired product.

The reaction mixture was washed three times with 125 ml of 3.0 M hydrochloric acid. Next, the mixture was phase separated and stripped with an aspirator for one hour at 40°C and under high vacuum for one hour at 55°C, giving a crude yield of 192.82 g. Analysis by gas chromatography showed a purity of 86.8%, corresponding to a corrected yield of 94.5% of the desired product. Distillation of a sample showed that the product contained 5.8 weight % non-volatile tars. The structure of the desired product was confirmed by mass spectroscopy.

### Comparison Example 1

This example illustrates the conduct of the process utilizing a cupric oxide/pyridine catalyst as in US—A—4,132,713.

In a flask there were mixed 200 g (0.586 mole) N-allyl-3'-trifluoromethyl-2,2-dichloroacetanilide, 8.25 g (0.058 mole) cupric oxide, 18.7 ml (0.023 mole) pyridine, and 135 ml toluene. The mixture was heated to reflux at 115°C and stirred for a total of one hour, 20 minutes. The mixture was filtered, then washed three times with 100 ml of 3.0M hydrochloric acid. Next, the mixture was phase separated and stripped first with an aspirator, then under high vacuum for one hour at 55°C to give a crude yield of 186.80 g. By gas chromatography, the reaction product was shown to be 80.0% pure, which corresponds to a corrected yield of 81.7% of the desired product. The product contained 14.8 weight % tars, as determined by distillation. The structure of the desired product was confirmed by mass spectroscopy.

### Example 2

In a flask were mixed 0.157 g (0.00158 mole) cuprous chloride, 1.6 ml (0.0091 mole) di(n-butyl)amine, and 40 ml toluene. The mixture was stirred to dissolve the cuprous chloride. Then, 11.22 g (0.0334 mole) of the acetanilide utilized in Example 1 was added, together with an additional 10 ml toluene. The resulting mixture was heated to 85—95°C and stirred for 1.5 hours.

At the end of this time the mixture was cooled, washed with 3M hydrochloric acid and stripped under vacuum, to produce 11.81 g of an orange oil, which was analyzed by mass spectroscopy and shown to be the desired pyrrolidone product. Gas chromatographic analysis of the product showed it to be 85.4% pure, which corresponds to a corrected yield of 96.7%. Distillation of a portion of the product showed it to contain 3.1% by weight of "tars".

### Example 3—12

The following examples represent conduct of the process according to this invention with other primary and secondary alkyl amines. These examples were all carried out by the following procedure.

The same acetanilide as in Examples 1 and 2 was mixed with 5 mole percent cuprous chloride and 28 mole percent of the indicated amine, utilizing tertiary butyl alcohol or toluene as the solvent. Reactions were carried out at approximately 85°C, or about 115°C when indicated, for the indicated time. Concentration of the acetanilide in the solvent was about 20—21 weight % except where indicated.

The reaction mixture was washed with dilute aqueous hydrochloric acid, the solvent removed and the products distilled. The reaction products were analyzed for percent by weight of the desired pyrrolidone, and of non-volatile side products ("tars"). Results of these experiments are shown in the following Table I.

TABLE I

| Example No. | Amine | Time, min. | Pyrrolidone Yield, %* | tar, wt. % |
|---|---|---|---|---|
| 3 | $HO(CH_2)_6NH_2$ | 60 | 86.7 | 5.4 |
| 4 | $i—C_4H_9NH_2$ | 120 | 84.5 | 4.4 |
| 5 | sec.—$C_4H_9NH_2$ | 120 | 88.2 | 4.4 |
| 6 | $(n—C_3H_7)_2NH$** | 120 | 90.3 | 5.7 |
| 7 | $(n—C_3H_7)_2NH$ | 85 | 88.6 | 5.2 |
| 8 | $(n—C_4H_9)_2NH$** | 170 | 91.3 | 6.2 |
| 9 | $(n—C_4H_9)_2NH$ | 60 | 90.6 | 7.6 |
| 10 | $(n—C_{12}H_{25})_2NH$ | 100 | 88.7 | 7.6 |
| 11 | $(n—C_{12}H_{25})_2NH$*** | 30 | 82.1 | 20.6 |
| 12 | $(n—C_{18}H_{37})_2NH$ | 75 | 89.3 | 24.2 |
| 13 | $(C_2H_5)_2NH$ | 60 | 84.8 | 4.2 |
| 14 | $(i—C_4H_9)_2NH$ | 150 | 93.3 | 3.6 |

* Corrected for unreacted starting material, if any.
** Toluene used as solvent.
*** Toluene used as solvent, temperature = 115°C, acetanilide concentration in solvent 61 weight %.

Comparison Example 2

The process was carried out as described for Examples 3—12 above with tertiary butyl alcohol, and cuprous chloride, at 85°C, but using pyridine as the amine rather than a primary or secondary amine. Results obtained were as follows:

| | |
|---|---|
| Pyrrolidone yield (corrected for unreacted starting material) | 89.0 wt. % |
| Tars (corrected for unreacted starting material) | 6.5 wt. % |
| Time for Reaction | 320 min. |

Thus, when using pyridine, a tertiary amine described in U.S. Patent 4,132,713, with the same amount of cuprous chloride as in Examples 3—14, completion of the reaction to achieve comparable yields required nearly 2.5 times as long as with the primary and secondary amines.

Examples 15—18

These examples demonstrate the conduct of the process utilizing cupric chloride in combination with lower and higher molecular weight amines. The process was carried out as above; experiments utilizing tertiary butyl alcohol as the solvent were conducted at 85°C, with an acetanilide concentration of 21 weight %, those utilizing toluene as the solvent were conducted at 115°C, with an acetanilide concentration of 61 weight %. Results are summarized in the following Table II.

TABLE II

| Example No. | Amine | Solvent | Time, min. | Pyrrolidone Yield, %* | tar, wt. % |
|---|---|---|---|---|---|
| 15 | $(n-C_4H_9)_2NH$ | t-butanol | 135 | 92.1 | 5.9 |
| 16 | $(n-C_{12}H_{25})_2NH$ | t-butanol | 110 | 84.4 | 15.7 |
| 17 | $(n-C_4H_9)_2NH$ | toluene | 60 | 85.5 | 12.1 |
| 18 | $(n-C_{12}H_{25})_2NH$ | toluene | 60 | 74.4 | 21.0 |

*Corrected for unreacted starting materials, if any.

The foregoing are intended to illustrate the conduct of the present process.

**Claims**

1. A process for the preparation of N-arylhalopyrrolidones having the formula

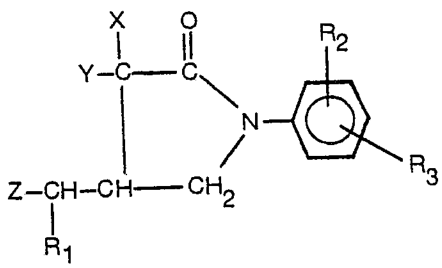

in which

X is hydrogen, chlorine, bromine or fluorine;
Y is hydrogen, chlorine, bromine or fluorine;
Z is chlorine or bromine
$R_1$ is hydrogen or $C_1$—$C_4$ alkyl;
$R_2$ is hydrogen, $C_1$—$C_4$ alkyl, acetyl, chlorine, bromine, fluorine, iodine, trifluoromethyl, nitro, cyano, $C_1$—$C_4$ alkoxy, $C_1$—$C_4$ alkylthio, $C_1$—$C_4$ alkylsulfinyl, $C_1$—$C_4$ alkylsulfonyl, trifluoromethylthio, trifluoromethylsulfinyl, trifluoromethylsulfonyl, pentafluoropropionamido or 3-methylureido; and
$R_3$ is hydrogen, $C_1$—$C_4$ alkyl, chlorine or trifluoromethyl,

by internal cyclization of the corresponding N-2-alkenyl-α-haloamide at a temperature of from 50 to 150°C in the presence of a copper-containing catalyst and an amine characterized by using an amine which is

(a) a primary amine having the formula $RNH_2$, in which R is a straight- or branched-chain alkyl group having from 1 to 20 carbon atoms, optionally substituted by hydroxy or

(b) a secondary amine having the formula $R_1NHR_2$, in which $R_1$ and $R_2$ are independently straight- or branched-chain alkyl groups having from 1 to 20 carbon atoms, optionally substituted by hydroxy exclusive of branched-chain alkyl groups having the branching at the alpha-carbon atom.

2. A process according to claim 1 in which a solvent is used, the N-2-alkenyl-α-haloamide is used in a concentration of 65 weight percent or less, the amine is used in an amount of 5 to 60 mole percent, based on the starting N-2-alkenyl-α-haloamide, and the copper containing catalyst is used in an amount of 0.1 to 20.0 mole percent, based on the starting N-2-alkenyl-α-haloamide.

3. A process according to claim 1 or 2 in which the copper catalyst comprises cuprous chloride.

4. A process according to any of claims 1—3 in which the temperature is from 70°C to 90°C.

5. A process according to any of claims 1—4 in which the copper catalyst comprises cupric oxide or cupric chloride.

6. A process according to any of claims 1—5 in which the amine is di(n-butyl)amino or di(n-propyl) amine.

7. A process according to any of claims 1—6 in which the copper catalyst is utilized in an amount of from 2.5 to 5 mole percent based on the α-haloamide.

8. A process according to any of claims 1—7 in which the time required to complete the reaction is from 30 to 180 minutes.

9. A process according to any of claims 1—8 in which X is chloro, Y is hydrogen, Z is chloro, $R_1$ is hydrogen, $R_2$ is hydrogen and $R_3$ is trifluoromethyl.

**Patentansprüche**

1. Verfahren zur Herstellung von N-Arylhalopyrrolidonen der Formel

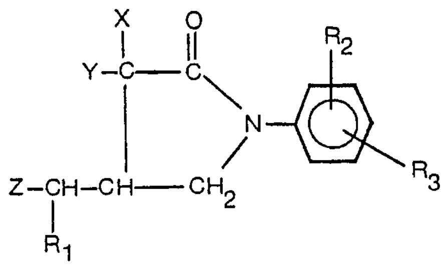

worin
X Wasserstoff, Chlor, Brom oder Fluor,
Y Wasserstoff, Chlor, Brom oder Fluor,
Z Chlor oder Brom
$R_1$ Wasserstoff oder eine Alkylgruppe mit 1—4 Kohlenstoffatomen,
$R_2$ Wasserstoff, ein Alkylrest mit 1—4 Kohlenstoffatomen, ein Acetylrest, Chlor, Brom, Fluor, Jod, eine Trifluormethylgruppe, eine Nitrogruppe, eine Cyanogruppe, ein Alkoxyrest mit 1—4 Kohlenstoffatomen, ein Thioalkylrest mit 1—4 Kohlenstoffatomen, eine Alkylsulfinylgruppe mit 1—4 Kohlenstoffatomen, eine Alkylsulfonylgruppe mit 1—4 Kohlenstoffatomen, eine Thiotrifluormethylgruppe, eine Trifluormethylsulfinylgruppe, eine Trifluormethylsulfonylgruppe, Pentafluorpropionamid oder 3-Methylureid und
$R_3$ Wasserstoff, ein Alkylrest mit 1—4 Kohlenstoffatomen, Chlor oder die Trifluormethylgruppe bedeuten, durch intramolekulare Cyclisierung des entsprechenden N-2-Alkenyl-α-haloamids bei einer Temperatur von 50 bis 150°C in Gegenwart eines Kupfer enthaltenden Katalysators und eines Amins,
dadurch gekennzeichnet, daß ein Amin eingesetzt wird, welches
a) ein primäres Amin der Formel $RNH_2$ ist, worin R eine verzweigte oder unverzweigte, walhweise hydroxysubstituierte Alkylgruppe mit 1—20 Kohlenstoffatomen ist oder
b) ein sekundäres Amin der Formel $R_1NHR_2$ ist, worin $R_1$und $R_2$ unabhängig voneinander verzweigte oder unverzweigte, wahlweise hydroxysubstituierte Alkylgruppen mit 1—20 Kohlenstoffatomen, jedoch keine verzweigte Alkylgruppen mit einer Verzweidung am α-Kohlenstoffatom sind.

2. Verfahren gemäß Anspruch 1, in dem ein Lösungsmittel verwendet wird, das N-2-Alkenyl-α-haloamid in einer Konzentration von 65 Gew.-% oder weniger eingesetzt wird, das Amin in einer Menge von 5—60 Molprozent, bezogen auf das Ausgangsmaterial N-2-Alkenyl-α-haloamid, und der Kupfer enthaltende Katalysator in einer Menge von 0,1 bis 20 Molprozent, bezogen auf das Ausgangsmaterial N-2-Alkenyl-α-haloamid, engesetzt wird.

3. Verfahren gemäß Anspruch 1 oder 2, worin der Kupfer enthaltende Katalysator Kupfer-I-chlorid enthält.

4. Verfahren gemäß einem der Ansprüche 1—3, dadurch gekennzeichnet, daß die Temperatur 70—90°C beträgt.

5. Verfahren gemäß einem der Ansprüche 1—4, wobei der Kupfer-Katalysator Kupfer-II-oxid oder Kupfer-II-chlorid enthält.

6. Verfahren gemäß einem der Ansprüche 1—5, wobei das Amin Di-n-Butylamin oder Di-n-Propylamin ist.

7. Verfahren gemäß einem der Ansprüche 1—6, dadurch gekennzeichnet, daß der Kupferkatalysator in einer Menge von 2,5 bis 5 Molprozent, bezogen auf das α-Haloamid verwendet wird.

8. Verfahren gemäß einem der Ansprüche 1—7, wobei die Reaktionszeit 30 bis 180 Minuten beträgt.

9. Verfahren gemäß einem der Ansprüche 1—8, wobei X Chlor, Y Wasserstoff, Z Chlor, $R_1$ Wasserstoff, $R_2$ Wasserstoff und $R_3$ die Trifluormethylgruppe bedeutet.

**Revendications**

1. Un procédé de préparation de N-arylhalopyrrolidones de formule:

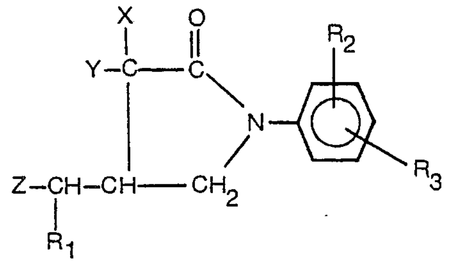

dans laquelle:

X représente un atome d'hydrogène, de chlore, de brome ou de fluor;

Y représente un atome d'hydrogène, de chlore, de brome ou de fluor;

Z représente un atome de chlore ou de brome;

$R_1$ représente un atome d'hydrogéne ou un radical alkyle en $C_1$ à $C_4$;

$R_2$ représente un atome d'hydrogène, un radical alkyle en $C_1$ à $C_4$, acétyle, un atome de chlore, de brome, de fluor, d'iode, un radical, trifluorométhyle, un groupe nitro, cyano, un radical alkoxy en $C_1$ à $C_4$, alkylthio en $C_1$ à $C_4$, alkylsulfinyle en $C_1$ à $C_4$, alkylsulfonyle en $C_1$ à $C_4$, trifluorométhylthio, trifluorométhylsulfinyle, trifluorométhylsulfonyle, pentafluoropropionamido ou 3-méthyluréido; et

$R_3$ représente un atome d'hydrogène, un radical alkyle en $C_1$ à $C_4$, un atome de chlore ou un radical trifluorométhyle,

par cyclisation interne du N-2-alkényle-α-haloamide correspondant, à une température de 50 à 150°C, en présence d'un catalyseur contenant du cuivre et d'une amine, caractérisé en ce que l'on utilise une amine qui est une:

(a) amine primaire de formule $RNH_2$ dans laquelle R représente un groupe alkyle à chaîne droite ou ramifiée renfermant de 1 à 20 atomes de carbone, éventuellement substitué par un groupe hydroxy; ou

(b) amine secondaire de formule $R_1NHR_2$ dans laquelle $R_1$ et $R_2$ représentent indépendamment des groupes alkyles à chaîne droite ou ramifiée renfermant de 1 à 20 atomes de carbone, éventuellement substitués par un groupe hydroxy, à l'exclusion des groupes alkyles à chaîne ramifiée dont la ramification se situe sur l'atome de carbone en alpha.

2. Un procédé selon la revendication 1, dans lequel on utilise un solvant, le N-2-alkényl-α-haloamide, utilisé en concentration de 65% en poids ou moins, l'amine est utilisée en quantité de 50 à 60 moles % par rapport au poids du N-2-alkényl-α-haloamide de départ, et le catalyseur contenant du cuivre est utilisé en quantité de 0,1 à 20,0 moles % par rapport au N-2-alkènyl-α-haloamide de départ.

3. Un procédé selon la revendication 1 ou 2, dans lequel le catalyseur à base de cuivre comprend du chlorure cuivreux.

4. Un procédé selon l'une quelconque des revendications 1 à 3, dans lequel la température est de 70 à 90°C.

5. Un procédé selon l'une quelconque des revendications 1 à 4, dans lequel le catalyseur à base de cuivre comprend de l'oxyde cuivrique ou du chlorure cuivrique.

6. Un procédé selon l'une quelconque des revendications 1 à 5, dans lequel l'amine est la di(n-butyl)amine ou la di(n-propyl)amine.

7. Un procédé selon l'une quelconque des revendications 1 à 6, dans lequel le catalyseur à base de cuivre est utilisé en quantité de 2,5 à 5 moles % par rapport à 1-α-haloamide.

8. Un procédé selon l'une quelconque des revendications 1 à 7, dans lequel le temps nécessaire pour achever la réaction est de 30 à 180 minutes.

9. Un procédé selon l'une quelconque des revendications 1 à 8, dans lequel X représente un atome de chlore, Y représente un atome d'hydrogène, Z représente un atome de chlore, $R_1$ représente un atome d'hydrogène, $R_2$ représente un atome d'hydrogène et $R_3$ représente un radical trifluorométhyle.